(19) Europäisches Patentamt
European Patent Office
Office européen des brevets

(11) Publication number : **0 450 232 B1**

(12) **EUROPEAN PATENT SPECIFICATION**

(45) Date of publication of patent specification : **30.03.94 Bulletin 94/13**

(51) Int. Cl.$^5$ : **A61K 31/40,** A61K 31/245

(21) Application number : **90313889.9**

(22) Date of filing : **19.12.90**

(54) **P-Guanidino benzoic acid derivatives for treatment of cerebrovascular contraction.**

(30) Priority : **05.04.90 JP 90682/90**

(43) Date of publication of application : **09.10.91 Bulletin 91/41**

(45) Publication of the grant of the patent : **30.03.94 Bulletin 94/13**

(84) Designated Contracting States : **AT CH DE FR GB IT LI NL**

(56) References cited :
**EP-A- 0 048 433**
**EP-A- 0 229 370**
**ASAIO TRANS., vol. 35, no. 3, 1988; T. AKIZAWA et al., pp. 176-178**
**J. APPL. PHYSIOL., vol. 67, no. 4, 1989; E. KREIL et al., pp. 1463-1471**

(73) Proprietor : **TORII & CO., LTD.**
**4-1, Nihonbashi-Honcho-3-chome**
**Chuo-ku Tokyo (JP)**

(72) Inventor : **Kikuchi, Haruhiko**
**22-8, Fujishirodai-2-chome**
**Suita-shi (JP)**
Inventor : **Yanamoto, Hiroji**
**12-19, Yoshida Ushinomiyacho**
**Sakyo-ku, Kyoto-shi (JP)**

(74) Representative : **Silveston, Judith et al**
**ABEL & IMRAY Northumberland House**
**303-306 High Holborn**
**London, WC1V 7LH (GB)**

**EP 0 450 232 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a medicinal agent effective for the treatment of cerebrovascular contraction caused by subarachnoid bleeding etc.

Subarachnoid bleeding occurs in about 12 persons per population of 100,000. About 50% of the patients will die or become disabled at the first attack and, if the patients receive no medical treatment, further 25-30% thereof will die owing to rebleeding.

Treatments commonly given at the onset of subarachnoid bleeding include rest in bed, control of blood pressure, administration of analgesics and sedatives, administration of hemostatic agents, control of encephalic pressure, and surgery.

At the subacute stage, further, cerebrovascular contraction takes place in about 40% of the patients. The cerebrovascular contraction at the subacute stage ranks second to rebleeding in importance regarding the prognosis of the patient. That is, the prognosis of the patient is greatly influenced by how well the cerebrovascular contraction can be suppressed after subarachnoid bleeding. No agent is known at present which can prevent or treat said cerebrovascular contraction. Accordingly, the invention of an agent which can prevent and/or treat cerebrovascular bleeding has been eagerly desired.

Compounds represented by the formula

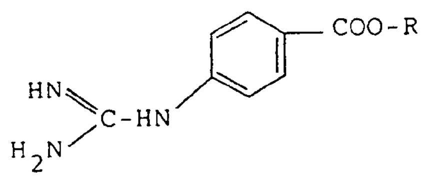

wherein R denotes a group represented by the formula

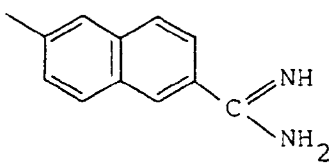

(A)

or a group represented by the formula

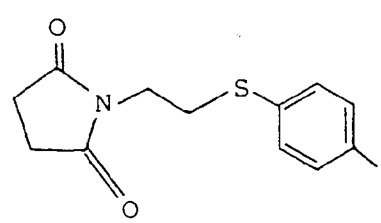

(B)

are already known. Compounds in which R represents formula (A) are disclosed in EP-A-48433 and compounds in which R represents formula (B) are disclosed in EP-A-229370. They are in use for the purpose of treating pancreatitis and they are agents whose safety and effectiveness are already known. However, it has not been known that these agents are effective in controlling cerebrovascular bleeding.

The present inventors have made extensive study to develop an agent which is effective in controlling cerebrovascular contraction subsequent to subarachnoid bleeding. As the result it has been found out that the compound represented by the formula

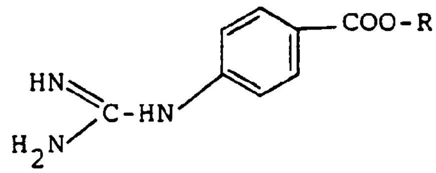

wherein R denotes a group represented by the formula

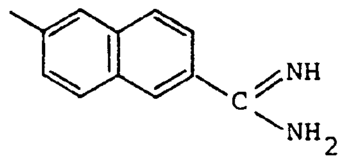

or a group represented by the formula

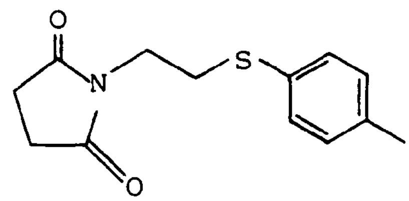

or a pharmaceutically acceptable salt thereof markedly improves the condition of cerebrovascular contraction occurring subsequently to subarachnoid bleeding.

The compound represented by the above formula may be administered by any methods conventionally used for the administration of medicinal agents, including injection, drip, and oral, rectal or sublingual administration. In an urgent need, however, it is desirably administered by intravenous injection.

The present invention provides the use of a p-guanidinobenzoic acid derivative of the formula defined above or a pharmaceutically acceptable salt thereof for the manufacture of a medicament for the prevention and/or treatment of cerebrovascular contraction and also for the manufacture of a medicament for the prevention and/or treatment of cerebrovascular bleeding.

The present invention will be described in detail below with reference to Examples.

By injecting a previously collected blood into the postcisterna of a rabbit, subarachnoid bleeding can be produced experimentally in the rabbit like human subarachnoid bleeding. The therapeutic effect of an agent is judged by comparing the thickness of the basal arteria before and after the experimental production of subarachnoid bleeding.

Example 1

Subarachnoid bleeding was produced experimentally in rabbits, and 20 minutes thereafter 3 mg, 6 mg and 9 mg of 6-amidino-2-naphthyl p-guanidinobenzoate dimethanesulfonate

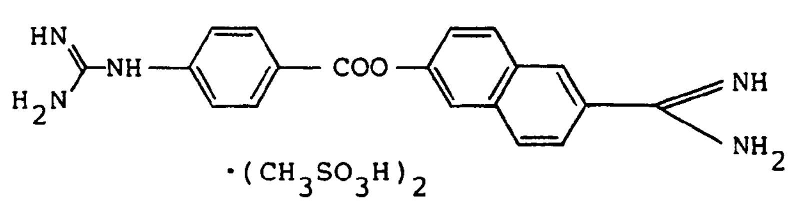

were respectively administered to the rabbits.

The ratios of the thickness of the basal arteria before subarachnoid bleeding to that after subarachnoid bleeding are shown in the Table.

Table

Effect of 6-amidino-2-naphthyl p-guanidinobenzoate dimethanesulfonate on experimental subarachnoid bleeding

| Dosage | Basal arteria thickness ratio (%) *) | | | | |
|---|---|---|---|---|---|
| | 1st day | 2nd day | 3rd day | 4th day | 5th day |
| 0 mg/kg | 73 | 65 | 71 | 78 | 84 |
| 3 mg/kg | 86 | 79 | 85 | 88 | 90 |
| 6 mg/kg | 97 | 95 | 95 | 96 | 101 |

Note:
*) Ratio of the thickness of basal arteria after subarachnoid bleeding to that before subarachnoid bleeding

The results shown in the Table reveal that 6-amidino-2-naphthyl p-guanidinobenzoate dimethanesulfonate has clearly suppressed the cerebrovascular contraction subsequent to subarachnoid bleeding.

Example 2

An experiment was performed in the same manner as in Example 1 except for using 4-(2-succinimidoethylthio)phenyl 4-guanidinobenzoate methanesulfonate

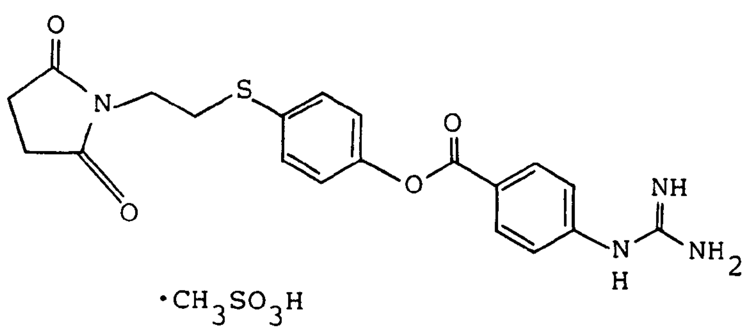

$\cdot CH_3SO_3H$

in place of 6-amidino-2-naphthyl p-guanidinobenzoate dimethanesulfonate. It was found that said compound also clearly suppressed the cerebrovascular contraction subsequent to subarachnoid bleeding, showing the same effect as that of the compound used in Example 1.

**Claims**

1. Use of a p-guanidinobenzoic acid derivative of the formula

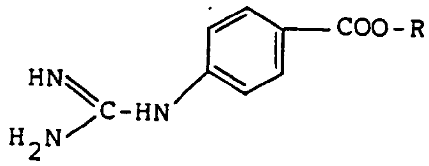

wherein R denotes a group represented by the formula

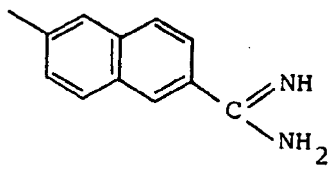

or a group represented by the formula

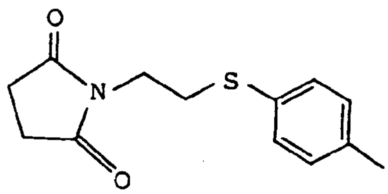

,

or a pharmaceutically acceptable salt thereof, for the manufacture of a medicament for the prevention and/or treatment of cerebrovascular contraction.

2. Use of a p-guanidinobenzoic acid derivative as defined in claim 1 or a pharmaceutically acceptable salt thereof for the manufacture of a medicament for the prevention and/or treatment of cerebrovascular bleeding.

## Patentansprüche

1. Verwendung eines p-Guanidinobenzoesäurederivates der Formel

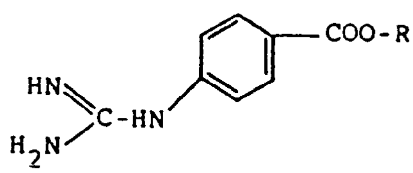

worin R für eine Gruppe der Formel

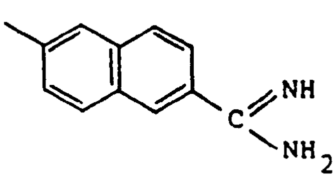

oder eine Gruppe der Formel

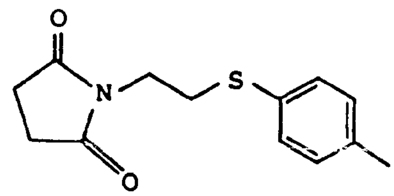

steht, oder eines pharmazeutisch akzeptablen Salzes davon zur Herstellung eines Medikamentes zur Prophylaxe und/oder Behandlung der cerebrovaskulären Kontraktion.

2. Verwendung eines wie in Anspruch 1 definierten p-Guanidinobenzoesäurederivates oder eines pharmazeutisch akzeptablen Salzes davon zur Herstellung eines Medikamentes zur Prophylaxe und/oder Behandlung der cerebrovaskulären Blutung.

## Revendications

1. Utilisation d'un dérivé d'acide p-guanidinobenzoïque de formule :

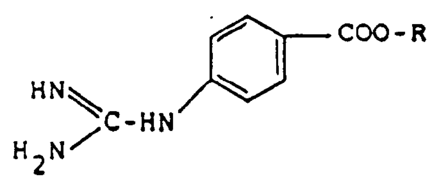

dans laquelle R représente un groupe de formule :

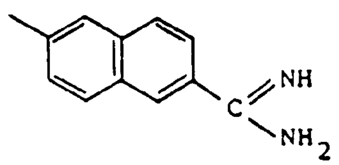

ou un groupe de formule :

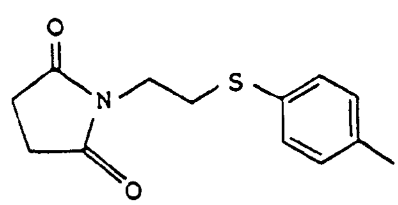

ou un sel pharmaceutiquement acceptable de celui-ci, pour la fabrication d'un médicament destiné à prévenir et/ou traiter une contraction cérébrovasculaire.

2. Utilisation d'un dérivé d'acide p-guanidinobenzoïque selon la revendication 1, ou d'un sel pharmaceutiquement acceptable de celui-ci, pour la fabrication d'un médicament destiné à prévenir et/ou traiter une hémorragie cérébrovasculaire.